# EUROPEAN PATENT SPECIFICATION

(11) **EP 3 342 459 B1**
(45) Date of publication and mention of the grant of the patent: **05.02.2020**
(21) Application number: 16850180.7
(22) Date of filing: 18.07.2016
(51) Int. Cl.: A61N 5/10

(54) **RADIATION DETECTION SYSTEM AND RADIATION DETECTION METHOD FOR NEUTRON CAPTURE THERAPY SYSTEM**
STRAHLUNGSDETEKTIONSSYSTEM UND STRAHLUNGSDETEKTIONSVERFAHREN FÜR NEUTRONENEINFANGTHERAPIESYSTEM
SYSTÈME DE DÉTECTION DE RAYONNEMENT ET PROCÉDÉ DE DÉTECTION DE RAYONNEMENT POUR SYSTÈME DE THÉRAPIE PAR CAPTURE DE NEUTRONS

(30) Priority: 28.09.2015 CN 201510629532; 28.09.2015 CN 201520760006 U
(43) Date of publication of application: 04.07.2018
(73) Proprietor: Neuboron Medtech Ltd., Nanjing, Jiangsu 211112 (CN)
(72) Inventor: LIU, Yuanhao, Nanjing Jiangsu 211112 (CN); CHEN, Weilin, Nanjing Jiangsu 211112 (CN)
(74) Representative: Sun, Yiming
(86) International application number: PCT/CN2016/090271
(87) International publication number: WO 2017/054556

(56) References cited:
- EP-A1- 0 313 716
- EP-A1- 2 805 745
- CN-A- 102 946 944
- CN-U- 205 073 541
- CN-U- 205 073 542
- TW-A- 201 438 788

## Description

### FIELD OF THE DISCLOSURE

The present disclosure relates generally to a radiation detection system and a radiation detection method, and, more particularly, to a radiation detection system and a radiation detection method for neutron capture therapy system.

### BACKGROUND OF THE DISCLOSURE

As atomics moves ahead, such radiotherapy as Cobalt-60, linear accelerators and electron beams has been one of major means to cancer therapy. However, conventional photon or electron therapy has been undergone physical restrictions of radioactive rays; for example, many normal tissues on a beam path will be damaged as tumor cells are destroyed. On the other hand, sensitivity of tumor cells to the radioactive rays differs greatly, so in most cases, conventional radiotherapy falls short of treatment effectiveness on radioresistant malignant tumors (such as glioblastoma multiforme and melanoma).

For the purpose of reducing radiation damage to the normal tissue surrounding a tumor site, target therapy in chemotherapy has been employed in the radiotherapy. While for high-radioresistant tumor cells, radiation sources with high RBE (relative biological effectiveness) including such as proton, heavy particle and neutron capture therapy have also developed. Among them, the neutron capture therapy combines the target therapy with the RBE, such as the boron neutron capture therapy (BNCT). By virtue of specific grouping of boronated pharmaceuticals in the tumor cells and precise neutron beam regulation, BNCT is provided as a better cancer therapy choice than conventional radiotherapy.

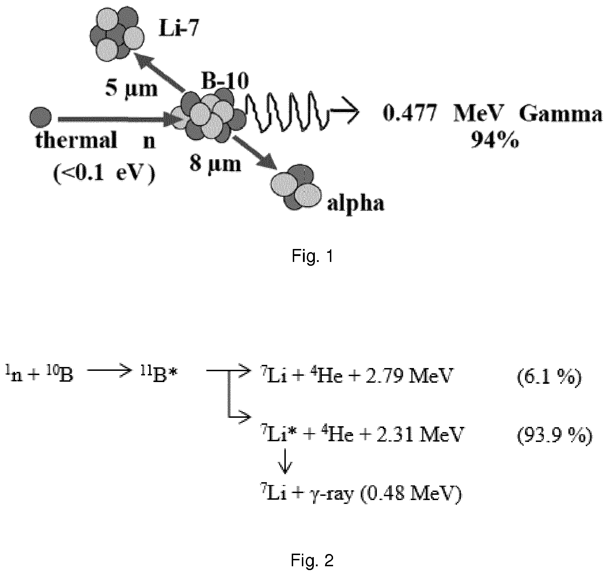

BNCT takes advantage that the boron (¹⁰B)-containing pharmaceuticals have high neutron capture cross section and produces ⁴He and ⁷Li heavy charged particles through ¹⁰B(n,α)⁷Li neutron capture and nuclear fission reaction. As illustrated in FIGS. 1 and 2, a schematic drawing of BNCT and a nuclear reaction formula of ¹⁰B(n,α)⁷Li neutron capture are shown, the two charged particles, with average energy at about 2.33MeV, are of linear energy transfer (LET) and short-range characteristics. LET and range of the alpha particle are 150keV/micrometer and 8 micrometers respectively while those of the heavy charged particle ⁷Li are 175keV/micrometer and 5 micrometers respectively, and the total range of the two particles approximately amounts to a cell size. Therefore, radiation damage to living organisms may be restricted at the cells' level. When the boronated pharmaceuticals are gathered in the tumor cells selectively, only the tumor cells will be destroyed locally with a proper neutron source on the premise of having no major normal tissue damage.

Beam detection and diagnosis which directly relates to the dose and effect of an irradiation therapy, belongs to an important subject in a neutron capture therapy system. As disclosed in the prior art, in a neutron capture therapy system, the dose of a neutron beam during irradiation is measured, for example, by attaching a gold wire for measuring a neutron beam to an irradiation object in advance, detaching the gold wire therefrom during the irradiation with a neutron beam, and measuring an amount of activated gold of the gold wire. It is intended to control (for example, stop) the neutron capture therapy system so as to irradiate the irradiation object with the neutron beam with a desired dose on the basis of the measured dose.

However, in this case, for example, when a dose rate of a neutron beam varies for some reasons after measuring the amount of activated gold of the gold wire, it may not be possible to cope with this variation and it may thus be difficult to irradiate an irradiation object with a neutron beam with a desired dose. That is to say, in the aforementioned neutron capture therapy system, the irradiation dose of the radiation cannot be detected in real time. Further, document TW 2014 38788 discloses a BNCT system comprising a γ ray detector located upstream of the neutron generating target as well as a neutron detector located in the neutron beam collimator.

Accordingly, it is necessary to provide a radiation detection system and a radiation detection method for neutron capture therapy system capable of improving the accuracy of a neutron beam irradiation dose.

### SUMMARY

One aspect of the present disclosure is to improve radiation detection system for neutron capture therapy system capable of improving the accuracy of a neutron beam irradiation dose. Wherein the neutron capture therapy system includes a charged particle beam, a charged particle beam inlet for passing the charged particle beam, a neutron generating unit generating a neutron beam by means of a nuclear reaction with the charged particle beam, and a beam shaping assembly for adjusting flux and quality of the neutron beam generated by the neutron generating unit and a beam outlet adjoining to the beam shaping assembly, wherein the neutron beam generating unit is arranged into the beam shaping assembly, and the radiation detection system includes a radiation detection device arranged outside the beam shaping assembly, the radiation detection device is used to detect the neutron beam overflowing from the neutron generating unit in real time after the charged particle beam and the neutron generating unit are subjected to the nuclear reaction.

Preferably, the radiation detection system further includes a control device, and wherein the control device sends a human-perceivable signal according to a detection result of the radiation detection system so as to confirm subsequent operation of the neutron capture therapy system. The human-perceivable signal may be a signal which can be perceived by human functional organs such as auditory sense, visual sense, tactile sense or smell sense. For example, the signal is one or more forms in various signals such as a sound-making alarm, an alarm lamp, a vibrating signal and a pungent smell emitting signal.

The neutron capture therapy system further includes an accelerator for accelerating the charged particle beam, and the control device includes a display unit for displaying the detection result of the radiation detection system and a control unit for feeding the detection result back to the accelerator so as to confirm subsequent operation of the accelerator, and the display unit may be common display equipment such as a television or a liquid crystal display.

More particularly, the radiation detection device is an ionization chamber or a scintillator, the radiation detection system calculates the intensity of the neutron beam according to detection signal, so that the charged particle beam is adjusted and the irradiation dose is controlled.

A common radiation detection device for real-time detection may have two different detection principles, namely an ionization chamber and a scintillator detector, can be realized. Those adopting ionization chamber structures as substrates include an He-3 proportional counter, a BF₃ proportional counter, a fission chamber and a boron ionization chamber. The scintillator detector may be divided into organic and inorganic materials, and for the purpose of detecting thermal neutrons, the scintillator detector mainly adds high thermal neutron capture section elements such as Li or B. In short, most of neutron energies detected by this type of detectors are the thermal neutrons, which are all heavy charged particles and nuclear fission fragments released by means of a capture or nuclear fission reaction between elements and neutrons, a great number of ion pairs are generated in the ionization chamber or the scintillator detector, and after the charges are collected, a current signal may be converted into a voltage pulse signal via appropriate circuit conversion. A neutron signal and a γ signal can be easily distinguished from each other by analyzing the magnitude of a voltage pulse. In a high-intensity neutron field such as a BNCT, the gas pressure of the ionization chamber, the concentration of coating of fissionable materials or boron or the concentration of the high neutron capture section elements in the scintillator detector can be appropriately reduced, so the sensitivity to neutrons can be effectively reduced, and the situation of signal saturation is avoided.

More preferably, the neutron beam detection system of present embodiment is a scintillator detector, and after certain materials absorb energy, visible light will be emitted, and the materials are referred to as scintillating materials. It utilizes ionizing radiation to excite an electron in a crystal or molecule to an exciting state, fluorescent light emitted when the electron returns to a base state is used for monitoring a neutron beam after being collected. The visible light emitted after the scintillator detector interacts with the neutron beam can be converted into an electron by utilizing a photomultiplier, and the electron is multiplied and amplified, wherein the multiplication and amplification rate of the electron can reach 10⁷ to 10⁸ usually. The quantity of electrons output from an anode is in direct proportion to energy of an incident neutron beam, and therefore the scintillator detector can measure the energy of the neutron beam.

The beam shaping assembly may include a reflector, a moderator surrounded by the reflector and adjoining to the neutron generating unit, a thermal neutron absorber adjoining to the moderator and a radiation shield arranged into the beam shaping assembly.

In another aspect of the present disclosure is to improve radiation detection method for neutron capture therapy system capable of improving the accuracy of a neutron beam irradiation dose. Wherein the neutron capture therapy system includes a charged particle beam, a charged particle beam inlet for passing the charged particle beam, a neutron generating unit generating a neutron beam by means of a nuclear reaction with the charged particle beam, and a beam shaping assembly for adjusting flux and quality of the neutron beam generated by the neutron generating unit and a beam outlet adjoining to the beam shaping assembly, wherein the neutron beam generating unit is arranged into the beam shaping assembly, and the radiation detection system includes a radiation detection device arranged outside the beam shaping assembly, the radiation detection device is used to detect the neutron beam overflowing from the neutron generating unit after the charged particle beam and the neutron generating unit are subjected to the nuclear reaction, and wherein the radiation detection method includes a detection step for detecting the neutron beam overflowing from or γ ray generated by the neutron generating unit in real time after the charged particle beam and the neutron generating unit are subjected to the nuclear reaction.

The radiation detection method further includes a controlling step for controlling subsequent operation of the neutron capture therapy system according to a detection result obtained in the detection step.

More particularly, the neutron capture therapy system further includes an accelerator for accelerating the charged particle beam, and the controlling step for controlling subsequent operation of the accelerator according to the detection result obtained in the detection step.

The radiation detection device further includes a display unit, the detection method includes a display step for displaying the detection result obtained in the detection step.

The detection method further includes a calculation step for calculating the intensity of the neutron beam according to detection signal, so that the charged particle beam is adjusted and the irradiation dose is controlled.

### BRIEF DESCRIPTION OF THE DRAWINGS

FIG. 1 is a schematic view of boron neutron capture reaction.
FIG. 2 is a nuclear reaction formula of ¹⁰B (n,α)⁷Li neutron capture.
FIG. 3 is a schematic view of the radiation detection system for neutron capture therapy system in one embodiment of the present disclosure.

### DETAILED DESCRIPTION

Neutron capture therapy (NCT) has been increasingly practiced as an effective cancer curing means in recent years, and BNCT is the most common. Neutrons for NCT may be supplied by nuclear reactors or accelerators. Take AB-BNCT for example, its principal components comprise, in general, an accelerator for accelerating charged particles (such as protons and deuterons), a target, a heat removal system and a beam shaping assembly. The accelerated charged particles interact with the metal target to produce the neutrons, and suitable nuclear reactions are always determined according to such characteristics as desired neutron yield and energy, available accelerated charged particle energy and current and materialization of the metal target, among which the most discussed two are ⁷Li (p, n)⁷Be and ⁹Be(p,n)⁹B and both are endothermic reaction. Their energy thresholds are 1.881 MeV and 2.055MeV respectively. Epithermal neutrons at a keV energy level are considered ideal neutron sources for BNCT. Theoretically, bombardment with lithium target using protons with energy slightly higher than the thresholds may produce neutrons relatively low in energy, so the neutrons may be used clinically without many moderations. However, Li (lithium) and Be (beryllium) and protons of threshold energy exhibit not high action cross section. In order to produce sufficient neutron fluxes, high-energy protons are usually selected to trigger the nuclear reactions.

The target, considered perfect, is supposed to have the advantages of high neutron yield, a produced neutron energy distribution near the epithermal neutron energy range (see details thereinafter), little strong-penetration radiation, safety, low cost, easy accessibility, high temperature resistance etc. But in reality, no nuclear reactions may satisfy all requests. The target in these embodiments of the present disclosure is made of lithium. However, well known by those skilled in the art, the target materials may be made of other metals besides the above-mentioned.

Requirements for the heat removal system differ as the selected nuclear reactions. ⁷Li(p, n)⁷Be asks for more than ⁹Be(p, n)⁹B does because of low melting point and poor thermal conductivity coefficient of the metal (lithium) target. In these embodiments of the present disclosure is ⁷Li(p, n)⁷Be.

No matter BNCT neutron sources are from the nuclear reactor or the nuclear reactions between the accelerator charged particles and the target, only mixed radiation fields are produced, that is, beams comprise neutrons and photons having energies from low to high. As for BNCT in the depth of tumors, except the epithermal neutrons, the more the residual quantity of radiation ray is, the higher the proportion of nonselective dose deposition in the normal tissue is. Therefore, radiation causing unnecessary dose should be lowered down as much as possible. Besides air beam quality factors, dose is calculated using a human head tissue prosthesis in order to understand dose distribution of the neutrons in the human body. The prosthesis beam quality factors are later used as design reference to the neutron beams, which is elaborated hereinafter.

The International Atomic Energy Agency (IAEA) has given five suggestions on the air beam quality factors for the clinical BNCT neutron sources. The suggestions may be used for differentiating the neutron sources and as reference for selecting neutron production pathways and designing the beam shaping assembly, and are shown as follows:
Epithermal neutron flux > 1 x 10⁹ n/cm²s
Fast neutron contamination < 2 x 10⁻¹³ Gy-cm²/n
Photon contamination < 2 x 10⁻¹³ Gy-cm²/n
Thermal to epithermal neutron flux ratio < 0.05
Epithermal neutron current to flux ratio > 0.7
Note: the epithermal neutron energy range is between 0.5eV and 40keV, the thermal neutron energy range is lower than 0.5eV, and the fast neutron energy range is higher than 40keV.

### 1. Epithermal neutron flux

The epithermal neutron flux and the concentration of the boronated pharmaceuticals at the tumor site codetermine clinical therapy time. If the boronated pharmaceuticals at the tumor site are high enough in concentration, the epithermal neutron flux may be reduced. On the contrary, if the concentration of the boronated pharmaceuticals in the tumors is at a low level, it is required that the epithermal neutrons in the high epithermal neutron flux should provide enough doses to the tumors. The given standard on the epithermal neutron flux from IAEA is more than 10⁹ epithermal neutrons per square centimeter per second. In this flux of neutron beams, therapy time may be approximately controlled shorter than an hour with the boronated pharmaceuticals. Thus, except that patients are well positioned and feel more comfortable in shorter therapy time, and limited residence time of the boronated pharmaceuticals in the tumors may be effectively utilized.

### 2. Fast neutron contamination

Unnecessary dose on the normal tissue produced by fast neutrons are considered as contamination. The dose exhibit positive correlation to neutron energy, hence, the quantity of the fast neutrons in the neutron beams should be reduced to the greatest extent. Dose of the fast neutrons per unit epithermal neutron flux is defined as the fast neutron contamination, and according to IAEA, it is supposed to be less than 2^{∗}10⁻¹³Gy-cm²/n.

### 3. Photon contamination (gamma-ray contamination)

Gamma-ray long-range penetration radiation will selectively result in dose deposit of all tissues in beam paths, so that lowering the quantity of gamma-ray is also the exclusive requirement in neutron beam design. Gamma-ray dose accompanied per unit epithermal neutron flux is defined as gamma-ray contamination which is suggested being less than 2^{∗}10⁻¹³Gy-cm²/n according to IAEA.

### 4. Thermal to epithermal neutron flux ratio

The thermal neutrons are so fast in rate of decay and poor in penetration that they leave most of energy in skin tissue after entering the body. Except for skin tumors like melanocytoma, the thermal neutrons serve as neutron sources of BNCT, in other cases like brain tumors, the quantity of the thermal neutrons has to be lowered. The thermal to epithermal neutron flux ratio is recommended at lower than 0.05 in accordance with IAEA.

### 5. Epithermal neutron current to flux ratio

The epithermal neutron current to flux ratio stands for beam direction, the higher the ratio is, the better the forward direction of the neutron beams is, and the neutron beams in the better forward direction may reduce dose surrounding the normal tissue resulted from neutron scattering. In addition, treatable depth as well as positioning posture is improved. The epithermal neutron current to flux ratio is better of larger than 0.7 according to IAEA.

The prosthesis beam quality factors are deduced by virtue of the dose distribution in the tissue obtained by the prosthesis according to a dose-depth curve of the normal tissue and the tumors. The three parameters as follows may be used for comparing different neutron beam therapy effects.

### 1. Advantage depth

Tumor dose is equal to the depth of the maximum dose of the normal tissue. Dose of the tumor cells at a position behind the depth is less than the maximum dose of the normal tissue, that is, boron neutron capture loses its advantages. The advantage depth indicates penetrability of neutron beams. Calculated in cm, the larger the advantage depth is, the larger the treatable tumor depth is.

### 2. Advantage depth dose rate

The advantage depth dose rate is the tumor dose rate of the advantage depth and also equal to the maximum dose rate of the normal tissue. It may have effects on length of the therapy time as the total dose on the normal tissue is a factor capable of influencing the total dose given to the tumors. The higher it is, the shorter the irradiation time for giving a certain dose on the tumors is, calculated by cGy/mA-min.

### 3. Advantage ratio

The average dose ratio received by the tumors and the normal tissue from the brain surface to the advantage depth is called as advantage ratio. The average ratio may be calculated using dose-depth curvilinear integral. The higher the advantage ratio is, the better the therapy effect of the neutron beams is.

To provide comparison reference to design of the beam shaping assembly, we also provide the following parameters for evaluating expression advantages and disadvantages of the neutron beams in the embodiments of the present disclosure except the air beam quality factors of IAEA and the abovementioned parameters.
1. Irradiation time <=30min (proton current for accelerator is 10mA)
2. 30.0RBE-Gy treatable depth >=7cm
3. The maximum tumor dose>=60.0RBE-Gy
4. The maximum dose of normal brain tissue<=12.5RBE-Gy
5. The maximum skin dose<=11.0RBE-Gy

Note: RBE stands for relative biological effectiveness. Since photons and neutrons express different biological effectiveness, the dose above should be multiplied with RBE of different tissues to obtain equivalent dose.

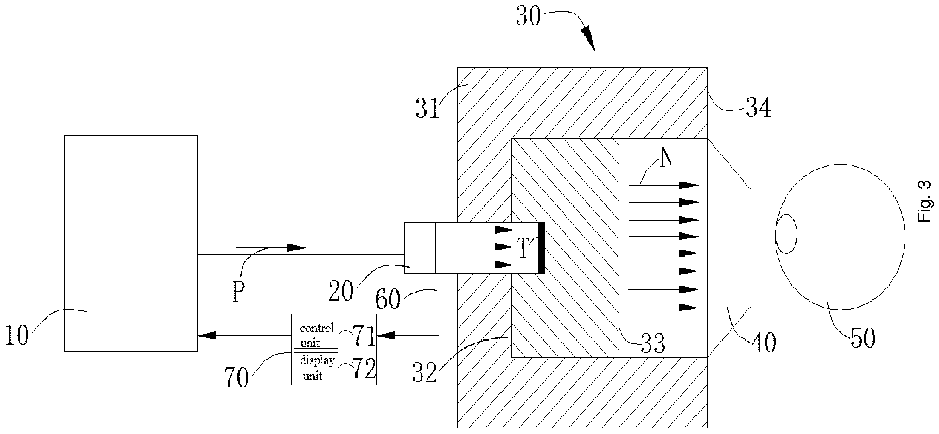

Referring to FIG. 3, one aspect of the embodiment aims at providing a radiation detection system used for improving the accuracy of the irradiation dosage of the neutron beam of a neutron capture therapy system. Another aspect of the embodiment provides a radiation detection method used for improving the accuracy of the irradiation dosage of the neutron beam of the neutron capture therapy system.

The neutron capture therapy system includes an accelerator 10, a beam expanding device 20, a charged particle beam inlet for passing a charged particle beam P, the charged particle beam P, a neutron beam generating unit T generating a neutron beam N by means of a nuclear reaction with the charged particle beam P, a beam shaping assembly 30 for adjusting flux and quality of the neutron beam generated by the neutron beam generating unit T, a beam outlet 40 adjoining to the beam shaping assembly 30, a irradiated body 50 irradiated by a beam emitted out from the beam outlet 40. The accelerator 10 is used for accelerating the charged particle beam P, and may be an accelerator suitable for an accelerator-type neutron capture therapy system such as a cyclotron or a linear accelerator. The charged particle beam P is preferably a proton beam. The beam expanding device 20 is disposed between the accelerator 10 and the neutron beam generating unit T. The charged particle beam inlet abuts the neutron beam generating unit T and is accommodated in the beam shaping assembly 30. Three arrows between the neutron beam generating unit T and the beam expanding device as shown in FIG. 3 serve as the charged particle beam inlet. The neutron beam generating unit T is accommodated in the beam shaping assembly 30. The neutron beam generating unit T is preferably lithium metal. The beam shaping assembly 30 includes a reflector 31, a moderator 32 which is surrounded by the reflector 31 and abuts the neutron beam generating unit T, a thermal neutron absorber 33 abutting the moderator 32, and a radiation shield 34 disposed in the beam shaping assembly 30. The neutron beam generating unit T and the charged particle beam P emitted from the charged particle beam inlet perform a nuclear reaction to generate the neutron beam N. The neutron beam defines a principal axis, the moderator 32 moderates neutrons generated by the neutron beam generating unit T to an epithermal neutron energy region, the reflector 31 guides the neutrons deviating from the principal axis back to the principal axis so as to improve the intensity of an epithermal neutron beam, the thermal neutron absorber 33 is used for absorbing thermal neutrons so as to avoid excess doses caused with normal tissues of a superficial layer during therapy, and the radiation shield 34 is used for shielding leaked neutrons and photons so as to reduce a normal tissue dose of a non-irradiation region. The beam outlet 40 may also be referred to as a neutron beam convergence part or a collimator, which reduces the widths of neutron beams so as to gather the neutron beams. The neutron beams emitted from the beam outlet 40 irradiate a target part of the irradiated body 50.

The radiation detection system used for improving the accuracy of the irradiation dosage of the neutron beam of the neutron capture therapy system includes a radiation detection device 60 arranged inside or outside the beam shaping assembly 30, and a control device 70. The radiation detection device 60 is used for detecting the neutron beam overflowing from the neutron generating unit T in real time after the charged particle beam P and the neutron generating unit T are subjected to a nuclear reaction. The control device 70 sends a human-perceivable signal according to a detection result of the radiation detection system so as to confirm subsequent operation of the neutron capture therapy system. The human-perceivable signal may be a signal which can be perceived by human functional organs such as auditory sense, visual sense, tactile sense or smell sense. For example, the signal is one or more forms in various signals such as a sound-making alarm, an alarm lamp, a vibrating signal and a pungent smell emitting signal.

The neutron capture therapy system further includes an accelerator for accelerating the charged particle beam, and the control device 70 includes a display unit 72 for displaying the detection result of the radiation detection system and a control unit 71 for feeding the detection result back to the accelerator so as to confirm subsequent operation of the accelerator, and the display unit may be common display equipment such as a television or a liquid crystal display.

A common radiation detection device for real-time detection may have two different detection principles, namely an ionization chamber and a scintillator detector, can be realized. Those adopting ionization chamber structures as substrates include an He-3 proportional counter, a BF₃ proportional counter, a fission chamber and a boron ionization chamber. The scintillator detector may be divided into organic and inorganic materials, and for the purpose of detecting thermal neutrons, the scintillator detector mainly adds high thermal neutron capture section elements such as Li or B. In short, most of neutron energies detected by this type of detectors are the thermal neutrons, which are all heavy charged particles and nuclear fission fragments released by means of a capture or nuclear fission reaction between elements and neutrons, a great number of ion pairs are generated in the ionization chamber or the scintillator detector, and after the charges are collected, a current signal may be converted into a voltage pulse signal via appropriate circuit conversion. A neutron signal and a γ signal can be easily distinguished from each other by analyzing the magnitude of a voltage pulse. In a high-intensity neutron field such as a BNCT, the gas pressure of the ionization chamber, the concentration of coating of fissionable materials or boron or the concentration of the high neutron capture section elements in the scintillator detector can be appropriately reduced, so the sensitivity to neutrons can be effectively reduced, and the situation of signal saturation is avoided.

More particularly, the neutron beam detection system in the embodiment adopts the ionization chamber. When passing through the ionization chamber, a neutron beam ionizes gas molecules inside the fission chamber or a wall portion of the ionization chamber to generate an electron and an ion with a positive charge, the electron and the positive charge ion are referred to as the aforementioned ion pair. The interior of the ionization chamber has an external electric field high voltage, so the electron moves toward a central anode wire, and the positive charge ion moves toward a surrounding cathode wall, thus generating an electronic pulse signal which can be measured. An energy needed for generating an ion pair by gas molecules is referred to as an average ionizing energy, the value varying with a gas type. For example, the average ionizing energy of air is about 34eV. If a neutron beam of 340keV exists, the air will generate about 10k ion pairs.

More preferably, the neutron beam detection system of another embodiment is a scintillator detector, and after certain materials absorb energy, visible light will be emitted, and the materials are referred to as scintillating materials. It utilizes ionizing radiation to excite an electron in a crystal or molecule to an exciting state, fluorescent light emitted when the electron returns to a base state is used for monitoring a neutron beam after being collected. The visible light emitted after the scintillator detector interacts with the neutron beam can be converted into an electron by utilizing a photomultiplier, and the electron is multiplied and amplified, wherein the multiplication and amplification rate of the electron can reach 10⁷ to 10⁸ usually. The quantity of electrons output from an anode is in direct proportion to energy of an incident neutron beam, and therefore the scintillator detector can measure the energy of the neutron beam or γ-ray.

The radiation detection system calculates the intensity of the neutron beam according to detection signal, so that the charged particle beam is adjusted and the irradiation dose is controlled.

As is well known by those skilled in the art that regardless of arrangement of the neutron beam detection system inside or adjacent to the beam shaping assembly, a detection device capable of detecting intensity variation and spatial distribution of the neutron beam in the beam shaping assembly can be adopted.

In another aspect of the present disclosure is to improve radiation detection method for neutron capture therapy system capable of improving the accuracy of a neutron beam irradiation dose. Wherein the neutron capture therapy system includes a charged particle beam, a charged particle beam inlet for passing the charged particle beam, a neutron generating unit generating a neutron beam by means of a nuclear reaction with the charged particle beam, and a beam shaping assembly for adjusting flux and quality of the neutron beam generated by the neutron generating unit and a beam outlet adjoining to the beam shaping assembly, wherein the neutron beam generating unit is arranged into the beam shaping assembly, and the radiation detection system includes a radiation detection device arranged inside or outside the beam shaping assembly, the radiation detection device is used to detect the neutron beam overflowing from the neutron generating unit after the charged particle beam and the neutron generating unit are subjected to the nuclear reaction, and wherein the radiation detection method includes a detection step for detecting the neutron beam overflowing from or γ ray generated by the neutron generating unit in real time after the charged particle beam and the neutron generating unit are subjected to the nuclear reaction.

The radiation detection method further includes a controlling step for controlling subsequent operation of the neutron capture therapy system according to a detection result obtained in the detection step.

More particularly, the neutron capture therapy system further includes an accelerator for accelerating the charged particle beam, and the controlling step for controlling subsequent operation of the accelerator according to the detection result obtained in the detection step.

The radiation detection device further includes a display unit, the detection method includes a display step for displaying the detection result obtained in the detection step. The detection method further includes a calculation step for calculating the intensity of the neutron beam according to detection signal, so that the charged particle beam is adjusted and the irradiation dose is controlled.

The invention is defined in the following claims, other embodiments being merely exemplary.

## Claims

1. A radiation detection system for neutron capture therapy system, wherein the neutron capture therapy system comprises:
an accelerator (10) for accelerating a charged particle beam (P);
a charged particle beam inlet for passing the charged particle beam;
a neutron beam generating unit (T) for generating a neutron beam (N) by means of a nuclear reaction with the charged particle beam;
a beam shaping assembly (30) for adjusting flux and quality of the neutron beam generated by the neutron beam generating unit; and
a beam outlet (40) adjoining to the beam shaping assembly,
wherein the neutron beam generating unit is arranged into the beam shaping assembly, and the radiation detection system comprises a radiation detection device (60), **characterized in that**
the radiation detection device is arranged upstream of the neutron beam generating unit;
the radiation detection device is configured to detect the neutron beam overflowing from the neutron generating unit in real time after the charged particle beam and the neutron generating unit are subjected to the nuclear reaction, and
a control device (70) is connected to the radiation detection device for controlling subsequent operation of the accelerator according to the detection result obtained by the radiation detection device.

2. The radiation detection system for neutron capture therapy system according to claim 1, wherein the control device is configured to send a human-perceivable signal according to a detection result of the radiation detection system so as to confirm subsequent operation of the neutron capture therapy system.

3. The radiation detection system for neutron capture therapy system according to claim 2, wherein the control device comprises a display unit (72) for displaying the detection result of the radiation detection system and a control unit (71) for feeding the detection result back to the accelerator so as to confirm subsequent operation of the accelerator.

4. The radiation detection system for neutron capture therapy system according to any one of claims 1 to 3, wherein the radiation detection device is an ionization chamber or a scintillator, the radiation detection system calculates the intensity of the neutron beam according to detection signal, so that the charged particle beam is adjusted and the irradiation dose is controlled.

5. The radiation detection system for neutron capture therapy system according to any one of claims 1 to 3, wherein the beam shaping assembly comprises a reflector (31), a moderator (32) surrounded by the reflector and adjoining to the neutron generating unit, a thermal neutron absorber (33) adjoining to the moderator and a radiation shield (34) arranged into the beam shaping assembly.

6. A radiation detection method for neutron capture therapy system, wherein the neutron capture therapy system comprises:
an accelerator (10) for accelerating a charged particle beam (P);
a charged particle beam inlet for passing the charged particle beam;
a neutron beam generating unit (T) for generating a neutron beam (N) by means of a nuclear reaction with the charged particle beam;
a beam shaping assembly (30) for adjusting flux and quality of the neutron beam generated by the neutron beam generating unit; and
a beam outlet (40) adjoining to the beam shaping assembly,
wherein the neutron beam generating unit is arranged into the beam shaping assembly, and the radiation detection system comprises a radiation detection device (60), **characterized in that**
the radiation detection device is arranged upstream of the neutron beam generating unit;
the radiation detection device is used to detect the neutron beam overflowing from the neutron generating unit after the charged particle beam and the neutron generating unit are subjected to the nuclear reaction, and
wherein the radiation detection method comprises a detection step for detecting the neutron beam overflowing from the neutron generating unit in real time after the charged particle beam and the neutron generating unit are subjected to the nuclear reaction, and
a controlling step for controlling subsequent operation of the accelerator according to the detection result obtained in the detection step.

7. The radiation detection method for neutron capture therapy system according to claim 6, wherein the controlling step comprises feeding the detection result back to the accelerator for controlling subsequent operation of the accelerator according to the detection result obtained in the detection step.

8. The radiation detection method for neutron capture therapy system according to any one of claims 6 and 7, wherein the radiation detection device comprises a display unit (72), the detection method comprises a display step for displaying the detection result obtained in the detection step.

9. The radiation detection method for neutron capture therapy system according to any one of claims 6 and 7, wherein the detection method comprises a calculation step for calculating the intensity of the neutron beam according to detection signal, so that the charged particle beam is adjusted and the irradiation dose is controlled.

## Patentansprüche

1. Strahlungserfassungssystem für ein Neutroneneinfangtherapiesystem, wobei das Neutroneneinfangtherapiesystem Folgendes umfasst:
einen Beschleuniger (10) zum Beschleunigen eines geladenen Partikelstrahls (P);
einen Einlass für den geladenen Partikelstrahl zum Hindurchführen des geladenen Partikelstrahls;
eine Neutronenstrahl erzeugende Einheit (T) zum Erzeugen eines Neutronenstrahls (N) durch eine Kernreaktion mit dem geladenen Partikelstrahl;
eine Strahlgestaltungsanordnung (30) zum Einstellen des Strömens und der Qualität des durch die Neutronenstrahl erzeugende Einheit erzeugten Neutronenstrahls; und
einen Strahlauslass (40), der an der Strahlgestaltungsanordnung anliegt,
wobei die Neutronenstrahl erzeugende Einheit in der Strahlgestaltungsanordnung angeordnet ist und das Strahlerfassungssystem eine Strahlerfassungsvorrichtung (60) umfasst,
**dadurch gekennzeichnet, dass**
die Strahlungserfassungvorrichtung stromaufwärts von der Neutronenstrahl erzeugenden Einheit angeordnet ist;
die Strahlungserfassungsvorrichtung zum Erfassen des Neutronenstrahls konfiguriert ist, der aus der Neutronenerzeugungseinheit in Echtzeit überströmt, nachdem der geladene Partikelstrahl und die Neutronenerzeugungseinheit der Kernreaktion unterworfen worden sind und
eine Kontrollvorrichtung (70) mit der Strahlungserfassungsvorrichtung zum Kontrollieren des darauffolgenden Arbeitsablaufs des Beschleunigers dem Erfassungsergebnis entsprechend, das durch die Strahlungserfassungsvorrichtung erhalten wird, verbunden ist.

2. Strahlungserfassungssystem für ein Neutroneneinfangtherapiesystem nach Anspruch 1, wobei die Kontrollvorrichtung konfiguriert ist, ein menschlich wahrnehmbares Signal einem Erfassungsergebnis des Strahlungserfassungssystems entsprechend zu senden, um den darauffolgenden Arbeitsgang des Neutroneneinfangtherapiesystems zu bestätigen.

3. Strahlungserfassungssystem für die Neutroneneinfangtherapie nach Anspruch 2, wobei die Kontrollvorrichtung eine Anzeigeeinheit (72) zum Anzeigen des Erfassungsergebnisses des Strahlungserfassungssystems und eine Kontrolleinheit (71) zum Zuführen des Erfassungsergebnisses zurück zu dem Beschleuniger umfasst, um den darauffolgenden Arbeitsablauf des Beschleunigers zu bestätigen.

4. Strahlungserfassungssystem für ein Neutroneneinfangtherapiesystem nach einem der Ansprüche 1 bis 3, wobei die Strahlungserfassungsvorrichtung eine lonisierungskammer oder ein Szintillator ist, das Strahlungserfassungssystem die Intensität des Neutronenstrahls dem Erfassungssignal entsprechend berechnet, so dass der geladene Partikelstrahl eingestellt und die Strahlungsdosis reguliert wird.

5. Strahlungserfassungssystem für ein Neutroneneinfangtherapiesystem nach einem der Ansprüche 1 bis 3, wobei die Strahlgestaltungsanordnung einen Reflektor (31), einen Moderator (32), der von dem Reflektor umgeben ist und an der Neutronenerzeugungseinheit anliegt, einen thermischen Neutronenabsorber (33), der an dem Moderator anliegt, und ein Strahlenschutzschild (34), das an der Strahlgestaltungsanordnung angeordnet ist, umfasst.

6. Strahlungserfassungsverfahren für ein Neutroneneinfangtherapiesystem, wobei das Neutroneneinfangtherapiesystem Folgendes umfasst:
einen Beschleuniger (10) zum Beschleunigen eines geladenen Partikelstrahls (P);
einen Einlass für den geladenen Partikelstrahl zum Hindurchführen des geladenen Partikelstrahls;
eine Neutronenstrahl erzeugende Einheit (T) zum Erzeugen eines Neutronenstrahls (N) durch eine Kernreaktion mit dem geladenen Partikelstrahl;
eine Strahlgestaltungsanordnung (30) zum Einstellen des Strömens und der Qualität des durch die Neutronenstrahl erzeugende Einheit erzeugten Neutronenstrahls; und
einen Strahlauslass (40), der an der Strahlgestaltungsanordnung anliegt,
wobei die Neutronenstrahl erzeugende Einheit in der Strahlgestaltungsanordnung angeordnet ist und das Strahlerfassungssystem eine Strahlerfassungsvorrichtung (60) umfasst,
**dadurch gekennzeichnet, dass**
die Strahlungserfassungvorrichtung stromaufwärts von der Neutronenstrahl erzeugenden Einheit angeordnet ist;
die Strahlungserfassungsvorrichtung zum Erfassen des Neutronenstrahls konfiguriert ist, der aus der Neutronenerzeugungseinheit überströmt, nachdem der geladene Partikelstrahl und die Neutronenerzeugungseinheit der Kernreaktion unterworfen worden sind und
wobei das Strahlungserfassungsverfahren einen Erfassungsschritt zum Erfassen des Neutronenstrahls umfasst, der aus der Neutronenerzeugungseinheit in Echtzeit überströmt, nachdem der geladene Partikelstrahl und die Neutronenerzeugungseinheit der Kernreaktion unterworfen worden sind und
einen Kontrollschritt zum Kontrollieren des darauffolgenden Arbeitsablaufs des Beschleunigers dem Erfassungsergebnis entsprechend, das im Erfassungsschritt erhalten wird.

7. Strahlungserfassungsverfahren für ein Neutroneneinfangtherapiesystem nach Anspruch 6, wobei der Kontrollschritt das Zuführen des Erfassungsergebnisses zurück zu dem Beschleuniger umfasst, um den darauffolgenden Arbeitsablauf des Beschleunigers dem Erfassungsergebnis, das im Erfassungsschritt erhalten wird, entsprechend zu kontrollieren.

8. Strahlungerfassungsverfahren für ein Neutroneneinfangtherapiesystem nach einem der Ansprüche 6 und 7, wobei die Strahlungserfassungsvorrichtung eine Anzeigeeinheit (72) umfasst, das Erfassungsverfahren einen Anzeigeschritt zum Anzeigen des Erfassungsergebnisses, das im Erfassungsschritt erhalten wird, umfasst.

9. Strahlungerfassungsverfahren für ein Neutroneneinfangtherapiesystem nach einem der Ansprüche 6 und 7, wobei das Erfassungsverfahren einen Berechnungsschritt zum Berechnen der Intensität des Neutronenstrahls dem Erfassungssignal entsprechend umfasst, derart, dass der geladene Partikelstrahl eingestellt und die Strahlungsdosis reguliert wird.

## Revendications

1. Système de détection de rayonnement destiné à un système de thérapie par capture de neutrons, dans lequel le système de thérapie par capture de neutrons comprend :
un accélérateur (10) permettant d'accélérer un faisceau de particules chargées (P) ;
une entrée de faisceau de particules chargées permettant de faire passer le faisceau de particules chargées ;
une unité de génération de faisceau de neutrons (T) permettant de générer un faisceau de neutrons (N) au moyen d'une réaction nucléaire avec le faisceau de particules chargées ;
un ensemble de mise en forme de faisceau (30) permettant de régler le flux et la qualité du faisceau de neutrons généré au moyen de l'unité de génération de faisceau de neutrons ; et
une sortie de faisceau (40) adjacente à l'ensemble de mise en forme de faisceau,
dans lequel l'unité de génération de faisceau de neutrons est agencée dans l'ensemble de mise en forme de faisceau, et le système de détection de rayonnement comprend un dispositif de détection de rayonnement (60),
**caractérisé en ce que**
le dispositif de détection de rayonnement est agencé en amont de l'unité de génération de faisceau de neutrons ;
le dispositif de détection de rayonnement est conçu pour détecter le faisceau de neutrons débordant de l'unité de génération de neutrons en temps réel après que le faisceau de particules chargées et l'unité de génération de neutrons soient soumis à la réaction nucléaire, et
un dispositif de commande (70) est relié au dispositif de détection de rayonnement pour le contrôle d'une opération ultérieure de l'accélérateur conformément au résultat de détection obtenu par le dispositif de détection de rayonnement.

2. Système de détection de rayonnement destiné à un système de thérapie par capture de neutrons selon la revendication 1, dans lequel le dispositif de commande est conçu pour envoyer un signal pouvant être perçu par un humain conformément à un résultat de détection du système de détection de rayonnement de manière à confirmer l'opération intérieure du système de thérapie par capture de neutrons.

3. Système de détection de rayonnement destiné à un système de thérapie par capture de neutrons selon la revendication 2, dans lequel le dispositif de commande comprend une unité d'affichage (72) permettant d'afficher le résultat de détection du système de détection de rayonnement et une unité de commande (71) permettant de renvoyer le résultat de détection à l'accélérateur de manière à confirmer l'opération ultérieure de l'accélérateur.

4. Système de détection de rayonnement destiné à un système de thérapie par capture de neutrons selon l'une quelconque des revendications 1 à 3, dans lequel le dispositif de détection de rayonnement et une chambre d'ionisation ou un scintillateur, le système de détection de rayonnement calcul intensité du faisceau de neutrons conformément au signal de détection, de sorte que le faisceau de particules chargées est ajusté et que la dose de rayonnement est contrôlée.

5. Système de détection de rayonnement destiné à un système de thérapie par capture de neutrons selon l'une quelconque des revendications 1 à 3, dans lequel l'ensemble de mise en forme de faisceau comprend un réflecteur (31), un modérateur (32) entouré du réflecteur et adjacent à l'unité de génération de neutrons, un absorbeur de neutrons thermiques (33) adjacent au modérateur et un écran anti-rayonnement (34) agencé dans l'ensemble de mise en forme de faisceau.

6. Procédé de détection de rayonnement destiné à un système de thérapie par capture de neutrons, dans lequel le système de thérapie par capture de neutrons comprend :
un accélérateur (10) permettant d'accélérer un faisceau de particules chargées (P) ;
une entrée de faisceau de particules chargées permettant de faire passer le faisceau de particules chargées ;
une unité de génération de faisceau de neutrons (T) permettant de générer un faisceau de neutrons (N) au moyen d'une réaction nucléaire avec le faisceau de particules chargées ;
un ensemble de mise en forme de faisceau (30) permettant d'ajuster le flux et la qualité du faisceau de neutrons généré par l'unité de génération de faisceau de neutrons ; et
une sortie de faisceau (40) adjacente à l'ensemble de mise en forme de faisceau,
dans lequel l'unité de génération de faisceau de neutrons est agencée dans l'ensemble de mise en forme de faisceau, et le système de détection de rayonnement comprend un dispositif de détection de rayonnement (60),
**caractérisé en ce que**
le dispositif de détection de rayonnement est agencé en amont de l'unité de génération de faisceau de neutrons ;
le dispositif de détection de rayonnement et le faisceau de neutrons débordant de l'unité de génération de neutrons après que le faisceau de particules chargées et l'unité de génération de neutrons soient soumis à la réaction nucléaire, et
dans lequel le procédé de détection de rayonnement comprend une étape de détection permettant de détecter le faisceau de neutrons débordant de l'unité de génération de neutrons en temps réel après que le faisceau de particules chargées et l'unité de génération de neutrons soient soumis à la réaction nucléaire, et
une étape de commande permettant de contrôler une opération ultérieure de l'accélérateur conformément au résultat de détection obtenu lors de l'étape de détection.

7. Procédé de détection de rayonnement destiné à un système de thérapie par capture de neutrons selon la revendication 6, dans lequel l'étape de commande comprend le renvoi du résultat de détection à l'accélérateur pour le contrôle d'une opération ultérieure de l'accélérateur conformément au résultat de détection obtenu lors de l'étape de détection.

8. Procédé de détection de rayonnement destiné à un système de thérapie par capture de neutrons selon la revendication 6 ou la revendication 7, dans lequel le dispositif de détection de rayonnement comprend l'unité d'affichage (72), le procédé de détection comprend une étape d'affichage permettant d'afficher le résultat de détection obtenu lors de l'étape de détection.

9. Procédé de détection de rayonnement destiné à un système de thérapie par capture de neutrons selon la revendication 6 ou la revendication 7, dans lequel le procédé de détection comprend une étape de calcul permettant de calculer l'intensité du faisceau de neutrons conformément au signal de détection, de sorte que le faisceau de particules chargées est ajusté et que la dose de rayonnement est contrôlée.
